(19) Europäisches Patentamt
European Patent Office
Office européen des brevets

(11) Veröffentlichungsnummer: **0 006 458**
**B1**

(12) **EUROPÄISCHE PATENTSCHRIFT**

(45) Veröffentlichungstag der Patentschrift: **07.10.81**

(51) Int. Cl.³: **C 07 C 103/82,** A 61 K 31/165, A 61 K 31/335, A 61 K 31/36, C 07 D 317/68, C 07 D 319/18

(21) Anmeldenummer: **79101634.8**

(22) Anmeldetag: **29.05.79**

(54) **Neue N1-Benzoyl-N2-phenyl-1,3-diaminopropan-2-ole und ihre Salze; Verfahren zu deren Herstellung und diese Verbindungen enthaltende Arzneimittel.**

(30) Priorität: **24.06.78 DE 2827801**

(43) Veröffentlichungstag der Anmeldung: **09.01.80 Patentblatt 80/1**

(45) Bekanntmachung des Hinweises auf die Patenterteilung: **07.10.81 Patentblatt 81/40**

(84) Benannte Vertragsstaaten: **AT BE CH DE FR GB IT LU NL SE**

(56) Entgegenhaltungen:
**DE-A-2 221 558**
**DE-A-2 336 399**
**DE-A1-2 720 908**
**DE-A1-2 720 968**

(73) Patentinhaber: **Kali-Chemie Pharma GmbH, Hans-Böckler-Allee 20 Postfach 220, D-3000 Hannover 1 (DE)**

(72) Erfinder: **Liepmann, Hans, Dipl.-Chem., Dr.rer.nat., Auf dem Emmerberge 17, D-3000 Hannover (DE)**
Erfinder: **Hüschens, Rolf, Dipl.-Chem., Dr.rer.nat., Matthäikirchstrasse 25, D-3000 Hannover 81 (DE)**
Erfinder: **Milkowski, Wolfgang, Dipl.-Chem., Dr. rer.nat., Fasanenweg 13, D-3167 Burgdorf (DE)**
Erfinder: **Zeugner, Horst, Dipl.-Chem., Dr.rer.nat., Havelweg 10, D-3000 Hannover 73 (DE)**
Erfinder: **Heinemann, Henning, Dipl.-Chem., Dr.rer.nat., Bergiusstrasse 18, D-3000 Hannover (DE)**
Erfinder: **Wolf, Klaus-Ullrich, Dr.vet., Imkersweg 6, D-3165 Hänigsen (DE)**
Erfinder: **Hell, Insa, Dr.vet., Wiesenstrasse 13, D-3000 Hannover (DE)**
Erfinder: **Hempel, Reinhard, Dr.med., Wolfsburger Damm 32, D-3000 Hannover 61 (DE)**

(74) Vertreter: **Lauer, Dieter, Dr. et al, c/o Kali-Chemie Aktiengesellschaft Postfach 220, D-3000 Hannover 1 (DE)**

**EP 0 006 458 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Neue $N_1$-Benzoyl-$N_2$-phenyl-1,3-diaminopropan-2-ole und ihre Salze; Verfahren zu deren Herstellung und diese Verbindungen enthaltende Arzneimittel

Die Erfindung betrifft neue $N_1$-Benzoyl-$N_2$-phenyl-1,3-diaminopropan-2-ole, deren pharmakologisch verträgliche Salze, Verfahren zu deren Herstellung sowie diese Substanzen als Wirkstoff enthaltende pharmazeutische Zusammensetzung.

Die $N_1$-Benzoyl-$N_2$-phenyl-1,3-diaminopropan-2-ole fallen zwar unter die allgemeine Formel II der DT–OS 2 221 558, sind aber dort nicht beschrieben. Gemäss dieser Schrift stellen sie wertvolle Zwischenprodukte zur Herstellung von Benzodiazepin- und Benzodiazocinderivaten dar, welche das Zentralnervensystem beeinflussen und aufgrund ihrer Eigenschaften als Tranquilizer, Sedativa oder Anticonvulsiva dienen können. Eine selbständige pharmakologische Wirkung ist für die Substanzen nicht beschrieben worden.

Überraschenderweise wurde gefunden, dass die neuen $N_1$-Benzoyl-$N_2$-phenyl-1,3-diaminopropan-2-ole der allgemeinen Formel I selbständige wertvolle pharmakologische und therapeutische Eigenschaften haben. Sie stabilisieren die gastrointestinale Schleimhaut gegenüber verschiedenen Schadwirkungen und eignen sich daher für die Therapie der akuten und chronischen Gastritis.

Die $N_1$-Benzoyl-$N_2$-phenyl-1,3-diaminopropan-2-ole der Formel I

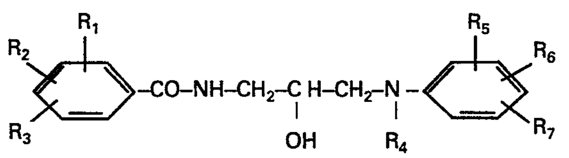

wobei $R_1$, $R_2$ und $R_3$ gleich und verschieden sein können und Hydroxygruppen, Benzyloxy-, Chlorbenzyloxygruppen oder Alkoxygruppen mit 1 bis 4 C-Atomen, wobei zwei benachbarte Gruppen eine Methylendioxy- oder Äthylendioxygruppe bilden können, bedeuten, oder wobei zwei Methoxygruppen mit einer Allyloxy-, Propargyloxy-, Acetoxy- oder Alkoxycarbonyloxygruppe, in welcher die Alkoxygruppe maximal 2 C-Atome haben kann, kombiniert sind,

$R_4$ eine Alkylgruppe mit 1 bis 4 C-Atomen, eine β-Hydroxyäthyl- oder β-Methoxyäthylgruppe oder Wasserstoffatom ist,

$R_5$, $R_6$ und $R_7$ gleich oder verschieden sein können und Wasserstoff- oder Halogenatome, Alkylgruppen mit 1 bis 4 C-Atomen, Alkoxygruppen mit 1 bis 4 C-Atomen, wobei zwei benachbarte Gruppen eine Methylendioxy- oder Äthylendioxygruppe bilden können, bedeuten, oder einer der Reste $R_5$, $R_6$ und $R_7$ eine Trifluormethyl- oder eine Nitrogruppe ist,

oder wenn $R_1$, $R_2$ und $R_3$ zusammen 3,4,5-Trimethoxy bedeuten und $R_4$ eine Methylgruppe ist, nicht mehr als zwei der Reste $R_5$, $R_6$ und $R_7$ die Bedeutung Wasserstoffatom besitzen, sowie deren Säureadditionssalze, oder wenn in den Verbindungen der allgemeinen Formel I mindestens einer der Reste $R_1$, $R_2$ und $R_3$ die Bedeutung Hydroxy hat, Salze dieser Verbindungen mit pharmakologisch verträglichen Kationen.

Als Alkylreste in den Alkyl- und Alkoxygruppen mit 1 bis 4 C-Atomen kommen gerade und verzweigte Alkylreste in Frage, wie Methyl, Äthyl, Propyl, Isopropyl, n-Butyl oder Isobutyl. Als Halogenatome eignen sich Fluor-, Chlor-, Brom- und Jodatome, insbesondere Fluor-, Chlor- und Bromatome.

Gegenstand der Erfindung sind ferner Verfahren zur Herstellung der Verbindungen der allgemeinen Formel I.

Die neuen Verbindungen werden dadurch erhalten, dass man

a) ein 1,3-Diaminopropan-2-ol der Formel II

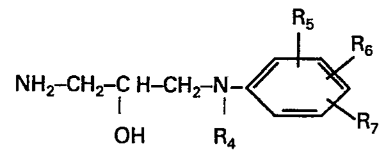

wobei $R_4$, $R_5$, $R_6$ und $R_7$ die oben angegebene Bedeutung haben, mit einem Benzoylderivat der Formel III

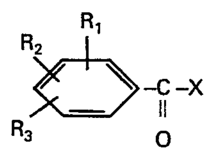

wobei $R_1$, $R_2$ und $R_3$ verätherte und/oder veresterte Hydroxygruppen der oben genannten Bedeutung sind und X ein reaktionsfähiger Säurerest, wie Halogen, eine niedermolekulare Alkoxygruppe oder O–CO–Y ist, worin Y eine niedermolekulare Alkoxygruppe bedeutet, ggf. in einem inerten Lösungsmittel bei Temperaturen zwischen − 10 °C und dem Siedepunkt des eingesetzten Lösungsmittels bei Normaldruck oder bei erhöhtem Druck umsetzt zur Verbindung der Formel I

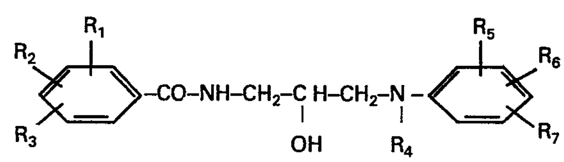

in welcher $R_1$, $R_2$ und $R_3$ verätherte und/oder veresterte Hydroxygruppen der oben genannten Bedeutung sind und $R_4$, $R_5$, $R_6$ und $R_7$ die obige Bedeutung haben,

b) gegebenenfalls einen oder mehrere der Substituenten $R_1$, $R_2$ und $R_3$ mit Bedeutung Benzyloxy oder Chlorbenzyloxy hydrogenolytisch zu den entsprechenden Hydroxygruppen spaltet,

c) gegebenenfalls Verbindungen mit den Substituenten $R_1$, $R_2$ oder $R_3$ mit Bedeutung Acetoxy oder Alkoxycarbonyloxy durch alkalische Hydrolyse in die entsprechenden Hydroxyverbindungen überführt,

d) gegebenenfalls einen oder mehrere der Substituenten $R_1$, $R_2$ und $R_3$ mit Bedeutung Hydroxy in die gewünschte Alkoxy-, Allyloxy-, Propargyloxy- oder in die gegebenenfalls substituierte Benzyloxygruppe der obigen Bedeutung umwandelt,
e) gegebenenfalls $R_4$ mit Bedeutung Wasserstoffatom in $R_4$ mit Bedeutung Alkyl umwandelt,
f) die isolierten freien Basen der Verbindungen der Formel I in die Säureadditionssalze überführt oder aus den Säureadditionssalzen die freien Basen gewinnt oder
g) gegebenenfalls die Verbindungen der Formel I mit pharmakologisch verträglichen Kationen zu den entsprechenden Metall-Oxidoverbindungen umsetzt, wenn mindestens einer der Reste $R_1$, $R_2$ und $R_3$ die Bedeutung Hydroxy hat.

Vorzugsweise wird die Umsetzung der 1,3-Diaminopropan-2-ole II mit den Benzoylderivaten der Formel III in Gegenwart eines säurebindenden Reagenzes, wie z.B. Kaliumcarbonat, Natriumcarbonat, Natriumhydroxid, Kaliumhydroxid, Triäthylamin oder Pyridin durchgeführt. Im Überschuss eingesetzt, können die tertiären Amine zusätzlich als inerte Lösungsmittel dienen. Als inerte Lösungsmittel eignen sich beispielsweise Methylenchlorid, Chloroform, Aceton, Tetrahydrofuran, Dioxan, Benzol, Toluol oder Chlorbenzol.

Zur Gewinnung von $N_1$-Benzoyl-$N_2$-phenyl-1,3-diaminopropan-2-olen der Formel I, in welchen ein oder mehrere der Substituenten $R_1$, $R_2$ und $R_3$ die Bedeutung Hydroxy haben, setzt man zweckmässigerweise 1,3-Diaminopropan-2-ole der Formel II mit Benzoylderivaten der Formel III um, in welchen die Hydroxygruppe mit einer Schutzgruppe versehen ist, wie beispielsweise einer Benzyl-, Acetyl- oder Alkoxycarbonylgruppe, und spaltet anschliessend die Schutzgruppe ab. Dieses lässt sich dadurch ermöglichen, dass man beispielsweise die Benzyloxygruppen hydrogenolytisch mit Wasserstoff in Gegenwart eines Edelmetallkatalysators, wie beispielsweise Palladiumkohle, bei Temperaturen zwischen 15 und 50 °C spaltet. Als Lösungsmittel können dabei niedere Alkohole, Dioxan, Tetrahydrofuran oder Äthylacetat dienen. Zur Spaltung der Acetoxy- oder Alkoxycarbonyloxygruppe kann man eine alkalische Hydrolyse mit beispielsweise Natrium- oder Kaliumhydroxid oder wässriger Ammoniaklösung bei Temperaturen zwischen 25 und 80 °C durchführen, wobei man als Lösungsmittel niedere Alkohole einsetzen und ggf. unter Inertgasatmosphäre, wie Stickstoff oder Wasserstoff arbeiten kann.

Aus den so erhaltenen $N_1$-Hydroxybenzoyl-$N_2$-phenyl-1,3-diaminopropan-2-olen der Formel I können ggf. auch die am Benzoylrest Äthergruppen enthaltenden Verbindungen der Formel I hergestellt werden, indem man die Alkalisalze der $N_1$-Hydroxybenzoylverbindungen in Gegenwart von niederen Alkoholen bei 30 bis 100 °C mit einem Halogenalkan, Halogenalken, Halogenalkin oder Halogenbenzyl, ggf. in einem geschlossenen Gefäss unter Schutzgasatmosphäre umsetzt.

Die nach diesem Verfahren gewonnenen Verbindungen der Formel I, in welchen $R_4$ die Bedeutung Wasserstoff hat, können durch nachträgliche Alkylierung in an sich bekannter Weise in die entsprechenden N-Alkylverbindungen, insbesondere N-Methyl- oder N-Äthylverbindungen, übergeführt werden. Dies geschieht beispielsweise nach den aus der Literatur bekannten Verfahren der reduktiven Carbonyl-Aminierung wie der Leuckart-Wallach- bzw. Eschweiler-Reaktion (s. H. Krauch, W. Kunz, Reaktionen der Organischen Chemie (1976), S. 126 und 131) oder durch Alkylierung mit Dialkylsulfaten (s. Houben-Weyl, XI/1 (1957), S. 207 ff).

Die aus dem Reaktionsgemisch isolierten freien Basen der Formel I können gewünschtenfalls durch Umsetzung mit anorganischen oder organischen Säuren nach an sich bekannten Methoden in ihre physiologisch verträglichen Säureadditionssalze übergeführt werden. Als geeignete Säuren haben sich z.B. Chlorwasserstoffsäure, Bromwasserstoffsäure, Schwefelsäure, Salpetersäure, Orthophosphorsäure, Cyclohexylaminosulfonsäure, Amidosulfonsäure oder p-Toluolsulfonsäure erwiesen.

Zu den pharmakologisch verträglichen Metalloxidoverbindungen der Formel I gelangt man, wenn man beispielsweise $N_1$-Hydroxybenzoyl-$N_2$-phenyl-1,3-diaminopropan-2-ole der Formel I ggf. unter Schutzatmosphäre, mit Alkali- oder Erdalkalialkoholaten in Gegenwart eines niederen Alkohols umsetzt oder mit einem Metallacetat bzw. einem Metallsalz, wie Metallchlorid oder Metallsulfat, in Eisessig/Wasser bei Temperaturen von 30–80 °C zur Umsetzung bringt. Als pharmakologisch verträgliche Metallsalze kommen z.B. die des Natrium, Magnesium, Zink, Kupfer, Aluminium oder Wismut in Frage.

Die 1,3-Diaminopropan-2-ole der Formel II können auf die von M. Chadwick et al. in J. Med. Chem. 9, S. 874 (1966) beschriebene Weise hergestellt werden.

Die erfindungsgemässen Verbindungen und ihre Salze zeigen neuartige pharmakologische Wirkungen. Von besonderem Interesse ist ihre Fähigkeit, die gastro-intestinale Schleimhaut gegenüber verschiedenen Schadwirkungen zu stabilisieren, wie sie zum Beispiel bei duodeno-gastrischem Reflux, bei Alkoholabusus und als Nebenwirkung in der Therapie mit nichtsteroidalen Antiphlogistika sowie Steroiden und Chemotherapeutika auftreten. Die Problematik einer gestörten Mucusbarriere haben z.B. W. F. Caspary in DMW 100 (1975) S. 1263–1268 und H. S. Murray et al. in Brit. Med. J. I, Nr. 5896, S. 19–21 (74) beschrieben.

Eine kausale medikamentöse Behandlung dieser Leiden ist zur Zeit unmöglich. Es werden daher symptomatisch Antazida eingesetzt.

Durch die besonderen pharmakologischen Eigenschaften der erfindungsgemässen Verbindungen wird erstmals eine kausale Therapie ermöglicht. Sie eignen sich für den klinischen Einsatz in der Behandlung der akuten und chronischen Gastritis.

Die oben zitierte Wirkung lässt sich durch den nachfolgenden pharmakologischen Test belegen.
Beschreibung der pharmakologischen Untersuchungsmethoden

1. Akute Toxizität

Die akute 7-Tage-Toxizität wird nach einmaliger Applikation per os an der weissen nüchternen NMRI-Maus bestimmt. Die Berechnung der $LD_{50}$-Werte erfolgt über EDV durch eine Probitanalyse (L. Cavalli-Sforza, Gustav Fischer-Verlag, Stuttgart (1964), Grundbegriffe der Biometrie, S. 153 ff.)

2. Magenzellverlust

Narkotisierte männliche Ratten des Stammes SIV 50 werden oral vor Versuchsbeginn mit der Testsubstanz behandelt. Anschliessend wird Acetylsalicylsäure als Irritans zur Provokation eines pathologisch erhöhten Epitelverlustes verabreicht. Zur Bestimmung des induzierten Gesamtzellverlustes am Rattenmagen wird den Tieren Acetylsalicylsäure ohne vorherige Gabe einer Schutzsubstanz verabreicht. Zur Präparation der Tiere für die Messung wird die Trachea freigelegt und intubiert. Es folgt eine Laparotomie in der Medianen, Vorlagerung des Magens und Einbindung eines Venenkatheters zur Entnahme von Magensaft. Durch den eingebundenen Katheter wird Magensaft entnommen, zentrifugiert und mikroskopisch ausgewertet.

Ermittelt wird die prozentuale Verringerung des durch Acetylsalicylsäure verursachten Zellverlustes nach vorheriger Verabreichung vom 3 mal 300 mg/kg der Testsubstanzen im Abstand von 12 h.

Als Testsubstanz wird beispielsweise eingesetzt:

| | |
|---|---|
| A) | $N_1$-(3,4,5-Trimethoxybenzoyl)-$N_2$-methyl-$N_2$-(4-fluorphenyl)-1,3-diaminopropan-2-ol |
| B) | $N_1$-(3,4,5-Trimethoxybenzoyl)-$N_2$-methyl-$N_2$-(4-chlorphenyl)-1,3-diaminopropan-2-ol |
| C) | $N_1$-(3,4,5-Trimethoxybenzoyl)-$N_2$-methyl-$N_2$-(4-trifluormethylphenyl)-1,3-diaminopropan-2-ol |
| D) | $N_1$-(3,4,5-Trimethoxybenzoyl)-$N_2$-methyl-$N_2$-(4-methylphenyl)-1,3-diaminopropan-2-ol |
| E) | $N_1$-(3,4,5-Trimethoxybenzoyl)-$N_2$-äthyl-$N_2$-(4-fluorphenyl)-1,3-diaminopropan-2-ol |
| F) | $N_1$-(3,4,5-Trimethoxybenzoyl)-$N_2$-(2,4,6-trimethylphenyl)-1,3-diaminopropan-2-ol |
| G) | $N_1$-(3,4,5-Triäthoxybenzoyl)-$N_2$-methyl-$N_2$-(4-fluorphenyl)-1,3-diaminopropan-2-ol |
| H) | $N_1$-(4-Methoxy-2,3-äthylendioxybenzoyl)-$N_2$-methyl-$N_2$-(4-fluorphenyl)-1,3-diaminopropan-2-ol |
| I) | $N_1$-(4-Butoxy-3,4-dimethoxybenzoyl)-$N_2$-methyl-$N_2$-(4-fluorphenyl)-1,3-diaminopropan-2-ol |

Als Standardsubstanz dient:

S) Aluminiumphosphat in Gelform.

Die Ergebnisse sind der nachfolgenden Tabelle zu entnehmen.

| Substanz | $LD_{50}$ p.o. (mg/kg) | % Minderung des Magenzellverlustes |
|---|---|---|
| A | 5010 | 53 |
| B | >5000 | 77 |
| C | >1390 | 47,8 |
| D | >3160 | 47,8 |
| E | >1280 | 43 |
| F | >1270 | 28,4 |
| G | >1370 | 63 |
| H | >1230 | 48,6 |
| I | >1370 | 27,2 |
| S | >5000 | 8,5 |

Die erfindungsgemässen Substanzen zeigen somit eine hervorragende Stabilisierung der Magenschleimhaut. Aufgrund ihrer geringen Toxizität besitzen sie zudem eine gute therapeutische Breite.

Die Verbindungen der allgemeinen Formel I und ihre Salze lassen sich in bekannter Weise in die üblichen pharmazeutischen Zubereitungsformen, z.B. in Lösungen, Suppositorien, Tabletten, Kapseln oder Dragées, einarbeiten. Die Einzeldosis beträgt für Erwachsene bei oraler Applikation 50 bis 150 mg und die Tagesdosis 150 bis 450 mg.

Beispiel I

Tabletten mit 100 mg ($N_1$-(3,4,5-Trimethoxybenzoyl)-$N_2$-methyl-$N_2$-(4-fluorphenyl)-1,3-diaminopropan-2-ol als Wirkstoff.

| 1 Tablette enthält: | |
|---|---|
| Wirkstoff | 100 mg |
| Lactose | 60 mg |
| Maisstärke | 30 mg |
| Primojel | 4 mg |
| Gelatine | 2 mg |
| Ärosil 200 | 2 mg |
| Magnesiumstearat | 2 mg |
| | 200 mg |

Herstellungsverfahren: Aus der Gelatine wird in Wasser ein 10%-iger Schleim hergestellt. Wirkstoff, Lactose, Maisstärke und Primojel werden gemischt und mit dem vorstehend hergestellten Schleim durch ein Sieb der Maschenweite 1,5 mm granuliert. Das Granulat wird bei 40 °C getrocknet, nochmals durch das Sieb passiert, mit Ärosil 200 und Magnesiumstearat vermischt und zu Tabletten verpresst. Stempel 9 mm.

Die nachstehenden Beispiele dienen zur Erläuterung der Erfindung.

Beispiel 1

Zu einer Lösung von 32,7 g $N_1$-Methyl-$N_1$-(4-fluorphenyl)-1,3-diaminopropan-2-ol und 18,1 g Triäthylamin in 400 ml Chloroform werden bei Raumtemperatur unter Rühren 38 g 3,4,5-Trimethoxybenzoylchlorid in 50 ml Chloroform zugetropft. Die Reaktionslösung wird nach 16 h mit Wasser versetzt, anschliessend die abgetrennte organische Phase mit Wasser gewaschen und über Natriumsulfat getrocknet. Der nach Filtrieren und Eindampfen des Lösungsmittels im Vakuum erhaltene Rückstand kristallisiert aus Aceton/Petroläther. Es werden 62,6 g $N_1$-(3,4,5-Trimethoxybenzoyl)-$N_2$-methyl-$N_2$-(4-fluorphenyl)-1,3-diaminopropan-2-ol vom Schmelzpunkt 113–116 °C erhalten.

Beispiel 2

Es werden 5,1 g 3,4,5-Trimethoxybenzoylchlorid in 100 ml Dioxan mit 4,0 g $N_1$-Methyl-$N_1$-(4-fluorphenyl)-1,3-diaminopropan-2-ol und 2,8 g Kaliumcarbonat 12 h gerührt. Die filtrierte Lösung wird im Vakuum abgezogen und der verbleibende Rückstand in 50 ml Methanol mit 15 ml 10%-iger Natriumhydroxidlösung 1 h auf 60 °C erwärmt. Das Lösungsmittel wird im Vakuum abgezogen. Die Substanz wird in Chloroform aufgenommen und isoliert. Aus Aceton/Petroläther werden 5,5 g kristallines $N_1$-(3,4,5-Trimethoxybenzoyl)-$N_2$-methyl-$N_2$-(4-fluorphenyl)-1,3-diaminopropan-2-ol mit Schmelzpunkt 113–116 °C erhalten.

Beispiel 3

4,0 g $N_1$-Methyl-$N_1$-(4-fluorphenyl)-1,3-diaminopropan-2-ol werden mit 5,1 g 3,4,5-Trimethoxybenzoylchlorid in 100 ml Benzol 6 h unter Rückfluss erhitzt. Anschliessend wird nach Zugabe von 20 ml 20%-iger wässriger Natriumhydroxidlösung 1,5 h bei etwa 60 °C gut durchmischt. Nach Aufarbeitung und Kristallisation aus Aceton/Petroläther erhält man 6,0 g $N_1$-(3,4,5-Trimethoxybenzoyl)-$N_2$-methyl-$N_2$-(4-fluorphenyl)-1,3-diaminopropan-2-ol mit Schmelzpunkt 113–116 °C.

Beispiel 4

Es werden 5,3 g 3,4,5-Trimethoxybenzoesäure in 40 ml Chloroform und 2,8 g Triäthylamin gelöst und bei 0–5 °C mit 3,0 g Chlorameisensäureäthylester versetzt. Nach 30 min. wird auf – 10 °C abgekühlt und eine Lösung von 5,0 g $N_1$-Methyl-$N_1$-(4-fluorphenyl)-1,3-diaminopropan-2-ol in 50 ml Chloroform zugegeben. Man lässt die Temperatur wieder auf 0–5 °C ansteigen. Nach 2 h wird die Lösung aufgearbeitet. Nach Chromatographie an Aluminiumoxid der Aktivitätsstufe II mit Chloroform/Toluol erhält man durch Kristallisation aus Aceton/Petroläther 2,8 g $N_1$-(3,4,5-Trimethoxybenzoyl-$N_2$-methyl-$N_2$-(4-fluorphenyl)-1,3-diaminopropan-2-ol mit Schmelzpunkt 113–116 °C.

Beispiel 5

1,9 g $N_1$-Methyl-$N_1$-(4-fluorphenyl)-1,3-diaminopropan-2-ol werden mit 2,3 g 3,4,5-Trimethoxybenzoesäuremethylester und 0,4 g gepulvertem Natriumhydroxid in 50 ml Xylol 1 h unter Rückfluss erhitzt. Nach Filtrieren und Eindampfen des Lösungsmittels werden 2,4 g $N_1$-(3,4,5-Trimethoxybenzoyl)-$N_2$-methyl-$N_2$-(4-fluorphenyl)-1,3-diaminopropan-2-ol erhalten. Die Substanz kristallisiert nach Filtration über Aluminiumoxid der Aktivitätsstufe II mit Toluol/Methylenchlorid aus Aceton/Petroläther mit einem Schmelzpunkt von 113–116 °C.

Beispiel 6

Entsprechend den Beispielen 1 bis 3 erhält man aus 4-Methoxy-3,5-dibenzyloxy-benzoylchlorid und $N_1$-Methyl-$N_1$-(4-chlorphenyl)-1,3-diaminopropan-2-ol das $N_1$-(4-Methoxy-3,5-dibenzyloxybenzoyl)-$N_2$-methyl-$N_2$-(4-chlorphenyl)-1,3-diaminopropan-2-ol mit Schmelzpunkt 155–158 °C.

Beispiel 7

Wird entsprechend den Reaktionsbedingungen der Beispiele 1 bis 5 ein 3,4,5-Trimethoxybenzoyl-Derivat der allgemeinen Formel II umgesetzt mit

---

1) N-Phenyl-1,3-diaminopropan-2-ol
2) N-(2-Fluorphenyl)-1,3-diaminopropan-2-ol
3) N-(3-Fluorphenyl)-1,3-diaminopropan-2-ol
4) N-(4-Fluorphenyl)-1,3-diaminopropan-2-ol
5) N-(2-Chlorphenyl)-1,3-diaminopropan-2-ol
6) N-(2-Methylphenyl)-1,3-diaminopropan-2-ol
7) N-(3-Methylphenyl)-1,3-diaminopropan-2-ol
8) N-(3,4-Dichlorphenyl)-1,3-diaminopropan-2-ol
9) N-(3-Chlor-2-methylphenyl)-1,3-diaminopropan-2-ol
10) N-(2,6-Dimethylphenyl)-1,3-diaminopropan-2-ol
11) $N_1$-Methyl-$N_1$-(4-chlorphenyl)-1,3-diaminopropan-2-ol
12) $N_1$-Methyl-$N_1$-(4-bromphenyl)-1,3-diaminopropan-2-ol
13) $N_1$-Methyl-$N_1$-(4-methylphenyl)-1,3-diaminopropan-2-ol
14) $N_1$-Methyl-$N_1$-(4-isopropylphenyl)-1,3-diaminopropan-2-ol
15) $N_1$-Methyl-$N_1$-(4-trifluormethylphenyl)-1,3-diaminopropan-2-ol
16) $N_1$-Methyl-$N_1$-(4-nitrophenyl)-1,3-diaminopropan-2-ol
17) $N_1$-Methyl-$N_1$-(3-methoxyphenyl)-1,3-diaminopropan-2-ol
18) $N_1$-Methyl-$N_1$-(3,4-dichlorphenyl)-1,3-diaminopropan-2-ol
19) $N_1$-Methyl-$N_1$-(3,4-dimethoxyphenyl)-1,3-diaminopropan-2-ol
20) $N_1$-Äthyl-$N_1$-phenyl-1,3-diaminopropan-2-ol
21) $N_1$-Äthyl-$N_1$-(4-fluorphenyl)-1,3-diaminopropan-2-ol
22) $N_1$-Propyl-$N_1$-phenyl-1,3-diaminopropan-2-ol
23) $N_1$-Propyl-$N_1$-(4-chlorphenyl)-1,3-diaminopropan-2-ol

24) $N_1$-Isopropyl-$N_1$-phenyl-1,3-diaminopropan-2-ol
25) $N_1$-(β-Hydroxyäthyl)-$N_1$-phenyl-1,3-diaminopropan-2-ol
26) $N_1$-(β-Methoxyäthyl)-$N_1$-(4-chlorphenyl)-1,3-diaminopropan-2-ol
27) N-(2,4,6-Trimethylphenyl)-1,3-diaminopropan-2-ol
28) $N_1$-Methyl-$N_1$-(4-äthylphenyl)-1,3-diaminopropan-2-ol

so erhält man

| | | Fp. °C |
|---|---|---|
| 1) | $N_1$-(3,4,5-Trimethoxybenzoyl)-$N_2$-phenyl-1,3-diaminopropan-2-ol | 163–168 |
| 2) | $N_1$-(3,4,5-Trimethoxybenzoyl)-$N_2$-(2-fluorphenyl)-1,3-diaminopropan-2-ol | 125–130 |
| 3) | $N_1$-(3,4,5-Trimethoxybenzoyl)-$N_2$-(3-fluorphenyl)-1,3-diaminopropan-2-ol | 155–159 |
| 4) | $N_1$-(3,4,5-Trimethoxybenzoyl)-$N_2$-(4-fluorphenyl)-1,3-diaminopropan-2-ol | 168–171 |
| 5) | $N_1$-(3,4,5-Trimethoxybenzoyl)-$N_2$-(2-chlorphenyl)-1,3-diaminopropan-2-ol | 106–108 |
| 6) | $N_1$-(3,4,5-Trimethoxybenzoyl)-$N_2$-(2-methylphenyl)-1,3-diaminopropan-2-ol | 122–125 |
| 7) | $N_1$-(3,4,5-Trimethoxybenzoyl)-$N_2$-(3-methylphenyl)-1,3-diaminopropan-2-ol | 137–139 |
| 8) | $N_1$-(3,4,5-Trimethoxybenzoyl)-$N_2$-(3,4-dichlorphenyl)-1,3-diaminopropan-2-ol | 158–161 |
| 9) | $N_1$-(3,4,5-Trimethoxybenzoyl)-$N_2$-(3-chlor-2-methylphenyl)-1,3-diaminopropan-2-ol | 135–136 |
| 10) | $N_1$-(3,4,5-Trimethoxybenzoyl)-$N_2$-(2,6-dimethylphenyl)-1,3-diaminopropan-2-ol | 81–84 |
| 11) | $N_1$-(3,4,5-Trimethoxybenzoyl)-$N_2$-methyl-$N_2$-(4-chlorphenyl)-1,3-diaminopropan-2-ol | 127–129 |
| 12) | $N_1$-(3,4,5-Trimethoxybenzoyl)-$N_2$-methyl-$N_2$-(4-bromphenyl)-1,3-diaminopropan-2-ol | 132–134 |
| 13) | $N_1$-(3,4,5-Trimethoxybenzoyl)-$N_2$-methyl-$N_2$-(4-methylphenyl)-1,3-diaminopropan-2-ol | 135–137 |
| 14) | $N_1$-(3,4,5-Trimethoxybenzoyl)-$N_2$-methyl-$N_2$-(4-isopropylphenyl)-1,3-diaminopropan-2-ol | 137–139 |
| 15) | $N_1$-(3,4,5-Trimethoxybenzoyl)-$N_2$-methyl-$N_2$-(4-trifluormethylphenyl)-1,3-diaminopropan-2-ol | 144–146 |
| 16) | $N_1$-(3,4,5-Trimethoxybenzoyl)-$N_2$-methyl-$N_2$-(4-nitrophenyl)-1,3-diaminopropan-2-ol | 172–175 |
| 17) | $N_1$-(3,4,5-Trimethoxybenzoyl)-$N_2$-methyl-$N_2$-(3-methoxyphenyl)-1,3-diaminopropan-2-ol | 148–150 |
| 18) | $N_1$-(3,4,5-Trimethoxybenzoyl)-$N_2$-methyl-$N_2$-(3,4-dichlorphenyl)-1,3-diaminopropan-2-ol | 140–143 |
| 19) | $N_1$-(3,4,5-Trimethoxybenzoyl)-$N_2$-methyl-$N_2$-(3,4-dimethoxyphenyl)-1,3-diaminopropan-2-ol | 69–74 |
| 20) | $N_1$-(3,4,5-Trimethoxybenzoyl)-$N_2$-äthyl-$N_2$-phenyl-1,3-diaminopropan-2-ol | 114–115 |
| 21) | $N_1$-(3,4,5-Trimethoxybenzoyl)-$N_2$-äthyl-$N_2$-(4-fluorphenyl)-1,3-diaminopropan-2-ol | 113–116 |
| 22) | $N_1$-(3,4,5-Trimethoxybenzoyl)-$N_2$-propyl-$N_2$-phenyl-1,3-diaminopropan-2-ol | 111–114 |
| 23) | $N_1$-(3,4,5-Trimethoxybenzoyl)-$N_2$-propyl-$N_2$-(4-chlorphenyl)-1,3-diaminopropan-2-ol | 140–142 |
| 24) | $N_1$-(3,4,5-Trimethoxybenzoyl)-$N_2$-isopropyl-$N_2$-phenyl-1,3-diaminopropan-2-ol | Öl*) |
| 25) | $N_1$-(3,4,5-Trimethoxybenzoyl)-$N_2$-(β-hydroxyäthyl)-$N_2$-phenyl-1,3-diaminopropan-2-ol | 100–102 |
| 26) | $N_1$-(3,4,5-Trimethoxybenzoyl)-$N_2$-(β-methoxyäthyl)-$N_2$-(4-chlorphenyl)-1,3-diaminopropan-2-ol | 105–108 |
| 27) | $N_1$-(3,4,5-Trimethoxybenzoyl)-$N_2$-(2,4,6-trimethylphenyl)-1,3-diaminopropan-2-ol | 102–104 |
| 28) | $N_1$-(3,4,5-Trimethoxybenzoyl)-$N_2$-methyl-$N_2$-(4-äthylphenyl)-1,3-diaminopropan -2-ol | 110–114 |

*) IR-Spektrum (Öl)$cm^{-1}$: 3350 (NH/OH); 1640 (NC=O)

Beispiel 8

Werden unter den Bedingungen der Beispiele 1 bis 3

1) 2,3,4-Trimethoxybenzoylchlorid,
2) 2,4,5-Trimethoxybenzoylchlorid
3) 3,4,5-Triäthoxybenzoylchlorid,
4) 4-Methoxy-3,5-dibenzyloxybenzoylchlorid,
5) 3,4,5-Tribenzyloxybenzoylchlorid,
6) 4-Acetoxy-3,5-dimethoxybenzoylchlorid
7) 4-Äthoxycarbonyloxy-3,5-dimethoxybenzoylchlorid,
8) 2-Methoxy-4,5-methylendioxybenzoylchlorid,
9) 4-Methoxy-2,3-äthylendioxybenzoylchlorid,
10) 4-Äthoxy-2,3-äthylendioxybenzoylchlorid,
11) 3-Methoxy-4,5-äthylendioxybenzoylchlorid bzw.
12) 3-Äthoxy-4,5-äthylendioxybenzoylchlorid

mit $N_1$-Methyl-$N_1$-(4-fluorphenyl)-1,3-diaminopropan-2-ol umgesetzt, so erhält man

| | | Fp. °C |
|---|---|---|
| 1) | $N_1$-(2,3,4-Trimethoxybenzoyl)-$N_2$-methyl-$N_2$-(4-fluorphenyl)-1,3-diaminopropan-2-ol | 84–85 |
| 2) | $N_1$-(2,4,5-Trimethoxybenzoyl)-$N_2$-methyl-$N_2$-(4-fluorphenyl)-1,3-diaminopropan-2-ol | 131–133 |
| 3) | $N_1$-(3,4,5-Triäthoxybenzoyl)-$N_2$-methyl-$N_2$-(4-fluorphenyl)-1,3-diaminopropan-2-ol | 90–95 |
| 4) | $N_1$-(4-Methoxy-3,5-dibenzyloxybenzoyl-$N_2$-methyl-$N_2$-(4-fluorphenyl-1,3-diaminopropan-2-ol | 137–139 |
| 5) | $N_1$-(3,4,5-Tribenzyloxybenzoyl)-$N_2$-methyl-$N_2$-(4-fluorphenyl)-1,3-diaminopropan-2-ol | 123–126 |
| 6) | $N_1$-(4-Acetoxy-3,5-dimethoxybenzoyl)-$N_2$-methyl-$N_2$-(4-fluorphenyl)-1,3-diaminopropan-2-ol | Öl*) |
| 7) | $N_1$-(4-Äthoxycarbonyloxy-3,5-dimethoxybenzoyl)-$N_2$-methyl-$N_2$-(4-fluorphenyl)-1,3-diaminopropan-2-ol,IR (Öl)$cm^{-1}$: 3360 (NH/OH), 1760(OC=O); 1640 (NC=O) | Öl |
| 8) | $N_1$-(2-Methoxy-4,5-methylendioxybenzoyl)-$N_2$-methyl-$N_2$-(4-fluorphenyl)-1,3-diaminopropan-2-ol, IR(Öl)$cm^{-1}$: 3380 (NH/OH);1640 (NC=O) | Öl |

*) IR (Öl)$cm^{-1}$: 3380 (NH/OH); 1765 (OC=O); 1640 (NC=O)

| | | |
|---|---|---|
| 9) | $N_1$-(4-Methoxy-2,3-äthylendioxybenzoyl)-$N_2$-methyl-$N_2$-(4-fluorphenyl)-1,3-diaminopropan-2-ol | 152–154 |
| 10) | $N_1$-(4-Äthoxy-2,3-äthylendioxybenzoyl)-$N_2$-methyl-$N_2$-(4-fluorphenyl)-1,3-diaminopropan-2-ol | 104–107 |
| 11) | $N_1$-(3-Methoxy-4,5-äthylendioxybenzoyl)-$N_2$-methyl-$N_2$-(4-fluorphenyl)-1,3-diaminopropan-2-ol | 118–120 |
| 12) | $N_1$-(3-Äthoxy-4,5-äthylendioxybenzoyl)-$N_2$-methyl-$N_2$-(4-fluorphenyl)-1,3-diaminopropan-2-ol | 151–157 |

Beispiel 9

2,1 g 2-Methoxy-4,5-methylendioxybenzoesäureäthylester werden mit 9,3 g $N_1$-Methyl-$N_1$-(4-fluorphenyl)-1,3-diaminopropan-2-ol und 0,4 g gepulvertem Natriumhydroxid unter Stickstoff 2 h auf 130 °C erhitzt. Das Reaktionsgemisch wird in Chloroform aufgenommen, vom Unlöslichen filtriert und das Filtrat an Aluminiumoxid der Aktivität II mit Chloroform chromatographiert. Es werden 1,2 g $N_1$-(2-Methoxy-4,5-methylendioxybenzoyl)-$N_2$-methyl-$N_2$-(4-fluorphenyl)-1,3-diaminopropan-2-ol aus Öl erhalten. IR-Spektrum (Öl) $cm^{-1}$: 3380 (NH/OH); 1640 (NC=O).

Beispiel 10

1,9 g $N_1$-(3,4,5-Trimethoxybenzoyl)-$N_2$-(4-fluorphenyl)-1,3-diaminopropan-2-ol werden mit 7,6 ml Ameisensäure und 2 ml 36%-iger wässriger Formalinlösung 3 h auf dem Wasserbad erhitzt. Die anschliessend mit Eis versetzte Reaktionslösung wird mit verdünnter Natriumhydroxidlösung alkalisch gestellt und die Substanz aus Chloroform isoliert. Nach Chromatographie an Aluminiumoxid der Aktivitätsstufe II mit Methylenchlorid/Chloroform werden nach Umkristallisieren aus Aceton/Petroläther 1,1 g $N_1$-(3,4,5-Trimethoxybenzoyl)-$N_2$-methyl-$N_2$-(4-fluorphenyl)-1,3-diaminopropan-2-ol vom Schmelzpunkt 113–116 °C erhalten.

Beispiel 11

1,9 g $N_1$-(3,4,5-Trimethoxybenzoyl)-$N_2$-(4-fluorphenyl)-1,3-diaminopropan-2-ol werden in 30 ml Dioxan gelöst, mit 1,5 g Natriumbicarbonat in 2,5 ml Wasser versetzt und nach Zugabe von 1,6 ml Dimethylsulfat 1 h auf 60 °C erwärmt. Anschliessend rührt man die Lösung mit 10 ml 15%-iger Natriumhydroxidlösung, zieht das Lösungsmittel im Vakuum ab und isoliert die Substanz aus Chloroform. Es werden 1,2 g $N_1$-(3,4,5-Trimethoxybenzoyl)-$N_2$-methyl-$N_2$-(4-fluorphenyl)-1,3-diaminopropan-2-ol erhalten. Die Substanz kristallisiert aus Aceton/Petroläther mit einem Schmelzpunkt von 113–116 °C.

Beispiel 12

3,6 g $N_1$-(3,4,5-Trimethoxybenzoyl)-$N_2$-phenyl-1,3-diaminopropan-2-ol werden in 60 ml Dioxan gelöst und mit 3,0 g Natriumbicarbonat in 6 ml Wasser versetzt. Nach Zugabe von 4,4 ml Diäthylsulfat wird 0,5 h auf 60 °C erwärmt. Anschliessend werden 5 ml 15%-ige Natriumhydroxidlösung zugegeben, das Lösungsmittel im Vakuum abgezogen und die Substanz aus Chloroform isoliert. Nach Chromatographie an Aluminiumoxid der Aktivitätsstufe II mit Methylenchlorid werden 2,5 g $N_1$-(3,4,5-Trimethoxybenzoyl)-$N_2$-äthyl-$N_2$-phenyl-1,3-diaminopropan-2-ol erhalten. Die aus Isopropanol kristallisierende Substanz schmilzt bei 114–115 °C.

Beispiel 13

24,8 g $N_1$-(3,4,5-Tribenzyloxybenzoyl)-$N_2$-methyl-$N_2$-(4-fluorphenyl)-1,3-diaminopropan-2-ol werden in 1000 ml Methanol gelöst und in Gegenwart von 2 g 5%-iger Palladiumkohle bei Normaldruck hydriert. Nach Abtrennen des Katalysators und des Lösungsmittels werden aus Äthylacetat/

Petroläther 13 g $N_1$-(3,4,5-Trihydroxybenzoyl)-$N_2$-methyl-$N_2$-(4-fluorphenyl)-1,3-diaminopropan-2-ol mit Fp. 167–169 °C kristallisiert.

Entsprechend werden aus 10,5 g $N_1$-(4-Methoxy-3,5-dibenzyloxybenzoyl)-$N_2$-methyl-$N_2$-(4-fluorphenyl)-1,3-diaminopropan-2-ol 6,3 g $N_1$-(4-Methoxy-3,5-dihydroxybenzoyl)-$N_2$-methyl-$N_2$-(4-fluorphenyl)-1,3-diaminopropan-2-ol mit Fp. 170–172 °C erhalten bzw. aus $N_1$-(4-Methoxy-3,5-dibenzyloxybenzoyl)-$N_2$-methyl-$N_2$-(4-chlorphenyl)-1,3-diaminopropan-2-ol das $N_1$-(4-Methoxy-3,5-dihydroxybenzoyl)-$N_2$-methyl-$N_2$-(4-chlorphenyl)-1,3-diaminopropan-2-ol als Öl. JR (Öl) $cm^{-1}$: 3200–3400 (NH/OH); 1625 (NC=O).

Beispiel 14

77 g $N_1$-(3,5-Dimethoxy-4-acetoxybenzoyl)-$N_2$-methyl-$N_2$-(4-fluorphenyl)-1,3-diaminopropan-2-ol werden in 400 ml Aceton gelöst und bei 50 °C mit 8,4 g Natriumhydroxid in 300 ml Wasser behandelt. Die Lösung wird mit verdünnter Salzsäure angesäuert, das Lösungsmittel im Vakuum abgezogen und die ausfallende Substanz abfiltriert. Es werden 55 g aus Isopropanol kristallisiertes $N_1$-(3,5-Dimethoxy-4-hydroxybenzoyl)-$N_2$-methyl-$N_2$-(4-fluorphenyl)-1,3-diaminopropan-2-ol vom Schmelzpunkt 161–164 °C erhalten. Das Hydrochlorid schmilzt bei 220–223 °C.

Beispiel 15

In entsprechender Weise erhält man aus 2 g $N_1$-(3,5-Dimethoxy-4-äthoxycarbonyloxybenzoyl)-$N_2$-methyl-$N_2$-(4-fluorphenyl)-1,3-diaminopropan-2-ol mit 5 ml wässriger Ammoniaklösung in 50 ml Methanol bei 70 °C in 2 h $N_1$-(3,5-Dimethoxy-4-hydroxybenzoyl)-$N_2$-methyl-$N_2$-(4-fluorphenyl)-1,3-diaminopropan-2-ol. Die aus Isopropanol kristallisierte Substanz schmilzt bei 161–164 °C.

Beispiel 16

3,8 g $N_1$-(4-Hydroxy-3,5-dimethoxybenzoyl)-$N_2$-methyl-$N_2$-(4-fluorphenyl)-1,3-diaminopropan-2-ol werden mit der äquivalenten Menge Natriummethylat (0,23 g Natrium in 50 ml Methanol) mit 6,0 g n-Butylbromid 16 h unter Rückfluss erhitzt. Das Reaktionsprodukt wird nach Abziehen des Lösungsmittels im Vakuum aus Chloroform isoliert und durch Filtration über Aluminiumoxid der Aktivitätsstufe II mit Chloroform gereinigt. Es werden 2,4 g aus Äther/Petroläther kristallisiertes $N_1$-(4-Butoxy-3,5-dimethoxybenzoyl)-$N_2$-methyl-$N_2$-(4-fluorphenyl)-1,3-diaminopropan-2-ol vom Schmelzpunkt 87–90 °C erhalten.

In entsprechender Weise erhält man aus $N_1$-(4-Hydroxy-3,5-dimethoxybenzoyl)-$N_2$-methyl-$N_2$-(4-fluorphenyl)-1,3-diaminopropan-2-ol und der äquivalenten Menge an Allylbromid, Propargylbromid oder 4-Chlorbenzylchlorid die Verbindungen $N_1$-(4-Allyloxy-3,5-dimethoxybenzoyl)-$N_2$-methyl-$N_2$-(4-fluorphenyl)-1,3-diaminopropan-2-ol vom Schmelzpunkt 100–103 °C, $N_1$-(4-Propargyloxy-3,5-dimethoxybenzoyl)-$N_2$-methyl-$N_2$-(4-fluorphenyl)-1,3-diaminopropan-2-ol vom Schmelzpunkt 135–138 °C und $N_1$-[4-(4-Chlorbenzyloxy)-3,5-dimethoxybenzoyl]-$N_2$-methyl-$N_2$-(4-fluorphenyl)-1,3-diaminopropan-2-ol vom Schmelzpunkt 140–144 °C.

Aus $N_1$-(4-Hydroxy-3,5-dimethoxybenzoyl)-$N_2$-methyl-$N_2$-(4-fluorphenyl)-1,3-diaminopropan-2-ol und der äquivalenten Menge Natriummethylat in Methanol und Überschuss an Isopropylbromid wird im Autoklav bei 90–95 °C $N_1$-(4-Isopropoxy-3,5-dimethoxybenzoyl)-$N_2$-methyl-$N_2$-(4-fluorphenyl)-1,3-diaminopropan-2-ol vom Schmelzpunkt 122–125 °C erhalten.

Beispiel 17

Zu 0,12 g Natrium in 50 ml Methanol werden 1,9 g $N_1$-(4-Hydroxy-3,5-dimethoxybenzoyl)-$N_2$-methyl-$N_2$-(4-fluorphenyl)-1,3-diaminopropan-2-ol gegeben und die Lösung 30 min. unter Rückfluss erhitzt. Der nach Abziehen des Lösungsmittels verbleibende Rückstand kristallisiert aus Isopropanol. Das Natrium-[$N_1$-(4-oxido-3,5-dimethoxybenzoyl)-$N_2$-methyl-$N_2$-(4-fluorphenyl)-1,3-diaminopropan-2-ol] schmilzt über 250 °C.

Beispiel 18

Zu einer Lösung von 0,079 g Magnesium in 50 ml Methanol wird eine Lösung von 2,46 g $N_1$-(4-Hydroxy-3,5-dimethoxybenzoyl)-$N_2$-methyl-$N_2$-(4-fluorphenyl)-1,3-diaminopropan-2-ol in 50 ml Methanol und 0,12 ml Wasser gegeben. Nach 2 h Erhitzen auf 65 °C erhält man Hydroxy-magnesium-[$N_1$-(4-oxido-3,5-dimethoxybenzoyl)-$N_2$-methyl-$N_2$-(4-fluorphenyl)-1,3-diaminopropan-2-ol] mit Schmelzpunkt über 250 °C.

Beispiel 19

1,3 g $N_1$-(4-Hydroxy-3,5-dimethoxybenzoyl)-$N_2$-methyl-$N_2$-(4-fluorphenyl)-1,3-diaminopropan-2-ol werden in 50 ml Wasser suspendiert. Nach Zugabe von verdünnter wässriger Natriumhydroxidlösung bis zur vollständigen Lösung wird eine Lösung von 0,8 g Kupfer(II)-sulfat-pentahydrat in 20 ml Wasser zugegeben und die Reaktionslösung 3 h bei Raumtemperatur belassen. Man erhält 1,5 g Hydroxy-kupfer(II)-[$N_1$-(4-oxido-3,5-dimethoxybenzoyl)-$N_2$-methyl-$N_2$-(4-fluorphenyl)-1,3-diaminopropan-2-ol] mit Schmelzpunkt über 250 °C.

In entsprechender Weise wird mit Zink(II)-sulfat-heptahydrat das Hydroxy-zink-[$N_1$-(4-oxido-3,5-dimethoxybenzoyl)-$N_2$-methyl-$N_2$-(4-fluorphenyl)-1,3-diaminopropan-2-ol] mit einem Schmelzpunkt über 200 °C erhalten.

Beispiel 20

2,3 g basisches Wismut(III)-nitrat und 4,0 g $N_1$-(3,4,5-Trihydroxybenzoyl)-$N_2$-methyl-$N_2$-(4-fluorphenyl)-1,3-diaminopropan-2-ol werden in einem Gemisch von 50 ml Essigsäure und 50 ml Wasser 2 h auf 65 °C erhitzt. Es werden 3,8 g Hydroxy-wismut(III)-[$N_1$-(3,4-dioxido-5-hydroxybenzoyl)-$N_2$-methyl-$N_2$-(4-fluorphenyl)-1,3-diaminopropan-2-ol] mit Schmelzpunkt über 200 °C erhalten.

In entsprechender Weise wird mit Aluminiumchlorid-hexahydrat das Hydroxy-aluminium-[$N_1$-(3,4-dioxido-5-hydroxybenzoyl)-$N_2$-methyl-$N_2$-(4-fluorphenyl)-1,3-diaminopropan-2-ol] vom Schmelzpunkt über 200 °C erhalten.

**Patentansprüche**

1. Neue $N_1$-Benzoyl-$N_2$-phenyl-1,3-diaminopropan-2-ole der Formel I

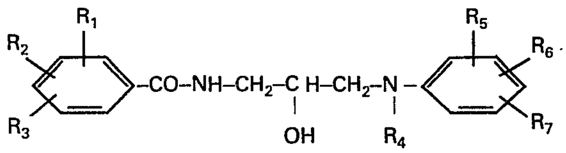

wobei $R_1$, $R_2$ und $R_3$ gleich und verschieden sein können und Hydroxygruppen, Benzyloxy-, Chlorbenzyloxygruppen oder Alkoxygruppen mit 1 bis 4 C-Atomen, wobei zwei benachbarte Gruppen eine Methylendioxy- oder Äthylendioxygruppe bilden können, bedeuten, oder wobei zwei Methoxygruppen mit einer Allyloxy-, Propargyloxy-, Acetoxy- oder Alkoxycarbonyloxygruppe, in welcher die Alkoxygruppe maximal 2 C-Atome haben kann, kombiniert sind,
$R_4$ eine Alkylgruppe mit 1 bis 4 C-Atomen, eine β-Hydroxyäthyl- oder β-Methoxyäthylgruppe oder Wasserstoffatom ist,
$R_5$, $R_6$ und $R_7$ gleich oder verschieden sein können und Wasserstoff- oder Halogenatome, Alkylgruppen mit 1 bis 4 C-Atomen, Alkoxygruppen mit 1 bis 4 C-Atomen, wobei zwei benachbarte Gruppen eine Methylendioxy- oder Äthylendioxygruppe bilden können, bedeuten, oder einer der Reste $R_5$, $R_6$ und $R_7$ eine Trifluormethyl- oder eine Nitrogruppe ist,
oder wenn $R_1$, $R_2$ und $R_3$ zusammen 3,4,5-Trimethoxy bedeuten und $R_4$ eine Methylgruppe ist, nicht mehr als zwei der Reste $R_5$, $R_6$ und $R_7$ die Bedeutung Wasserstoffatom besitzen, sowie deren Säureadditionssalze, oder wenn in den Verbindungen der allgemeinen Formel I mindestens einer der Reste $R_1$, $R_2$ und $R_3$ die Bedeutung Hydroxy hat, Salze dieser Verbindungen mit pharmakologisch verträglichen Kationen.

2. $N_1$-(3,4,5-Trimethoxybenzoyl)-$N_2$-A-1,3-diaminopropan-2-ol entsprechend Anspruch 1, in welchem A ein Phenyl-, 2-Fluorphenyl-, 3-Fluorphenyl-, 4-Fluorphenyl-, 2-Chlorphenyl-, 2-Methylphenyl-, 3-Methylphenyl-, 3,4-Dichlorphenyl-, 3-Chlor-2-methylphenyl-, 2,6-Dimethylphenyl- oder 2,4,6-Trimethylphenylrest ist.

3. $N_1$-(3,4,5-Trimethoxybenzoyl)-$N_2$-methyl-$N_2$-A-1,3-diaminopropan-2-ol entsprechend Anspruch 1, in welchem A ein 4-Fluorphenyl-, 4-Chlorphenyl-, 4-Bromphenyl-, 4-Methylphenyl-, 4-Äthylphenyl-, 4-Isopropylphenyl-, 4-Trifluormethylphenyl-, 3-Methoxyphenyl-, 3,4-Dichlorphenyl- oder 3,4-Dimethoxyphenyl- oder 4-Nitrophenylrest ist.

4. $N_1$-(3,4,5-Trimethoxybenzoyl)-$N_2$-B-$N_2$-phenyl-1,3-diaminopropan-2-ol, entsprechend Anspruch 1, in welchem B ein Äthyl-, Propyl-, Isopropyl- oder β-Hydroxyäthylrest ist.

5. $N_1$-(3,4,5-Trimethoxybenzoyl)-$N_2$-B-$N_2$-(4-chlorphenyl)-1,3-diaminopropan-2-ol entsprechend Anspruch 1, in welchem B ein Propyl- oder β-Methoxyäthylrest ist.

6. $N_1$-(3,4,5-Trimethoxybenzoyl)-$N_2$-äthyl-$N_2$-(4-fluorphenyl)-1,3-diaminopropan-2-ol.

7. $N_1$-C-$N_2$-methyl-$N_2$-(4-fluorphenyl)-1,3-diaminopropan-2-ol entsprechend Anspruch 1, in welchem C ein 2,3,4-Trimethoxybenzoyl-, 2,4,5-Trimethoxybenzoyl-, 3,4,5-Triäthoxybenzoyl-, 4-Methoxy-3,5-dibenzyloxybenzoyl-, 3,4,5-Tribenzyloxybenzoyl-, 4-Acetoxy-3,5-dimethoxybenzoyl-, 4-Äthoxycarbonyloxy-3,5-dimethoxybenzoyl-, 4-Hydroxy-3,5-dimethoxybenzoyl-, 4-Methoxy-3,5-dihydroxybenzoyl-, 3,4,5-Trihydroxybenzoyl-, 4-Isopropoxy-3,5-dimethoxybenzoyl-, 4-Butoxy-3,5-dimethoxybenzoyl-, 4-Allyloxy-3,5-dimethoxybenzoyl-, 4-Propargyloxy-3,5-dimethoxybenzoyl-, 4-(4-Chlorbenzyloxy)-3,5-dimethoxybenzoyl-,2-Methoxy-4,5-methylendioxybenzoyl-, 4-Methoxy-2,3-äthylendioxybenzoyl-, 4-Äthoxy-2,3-äthylendioxybenzoyl-, 3-Methoxy-4,5-äthylendioxybenzoyl-, 3-Äthoxy-4,5-äthylendioxybenzoylrest ist.

8. $N_1$-C-$N_2$-methyl-$N_2$-(4-chlorphenyl)-1,3-diaminopropan-2-ol entsprechend Anspruch 1 in welchem C 4-Methoxy-3,5-dibenzyloxybenzoyl- oder 4-Methoxy-3,5-dihydroxybenzoylrest ist.

9. Verfahren zur Herstellung von $N_1$-Benzoyl-$N_2$-phenyl-1,3-diaminopropan-2-olen der Formel I

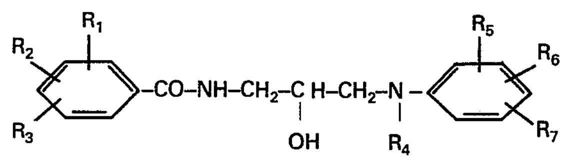

wobei $R_1$, $R_2$ und $R_3$ gleich und verschieden sein können und Hydroxygruppen, Benzyloxy-, Chlorbenzyloxygruppen oder Alkoxygruppen mit 1 bis 4 C-Atomen, wobei zwei benachbarte Gruppen eine Methylendioxy- oder Äthylendioxygruppe bilden können, bedeuten, oder wobei zwei Methoxygruppen mit einer Allyloxy-, Propargyloxy-, Acetoxy- oder Alkoxycarbonyloxygruppe, in welcher die Alkoxygruppe maximal 2 C-Atome haben kann, kombiniert sind,
$R_4$ eine Alkylgruppe mit 1 bis 4 C-Atomen, eine β-Hydroxyäthyl- oder β-Methoxyäthylgruppe oder Wasserstoffatom ist,
$R_5$, $R_6$ und $R_7$ gleich oder verschieden sein können und Wasserstoff- oder Halogenatome, Alkylgruppen mit 1 bis 4 C-Atomen, Alkoxygruppen mit 1 bis 4 C-Atomen, wobei zwei benachbarte Gruppen eine Methylendioxy- oder Äthylendioxygruppe bilden können, bedeuten, oder einer der Reste $R_5$, $R_6$ und $R_7$ eine Trifluormethyl- oder eine Nitrogruppe ist, oder wenn $R_1$, $R_2$ und $R_3$ zusammen 3,4,5-Trimethoxy bedeuten und $R_4$ eine Methylgruppe ist, nicht mehr als zwei der Reste $R_5$, $R_6$ und $R_7$ die Bedeutung Wasserstoffatom besitzen, sowie deren Säureadditionssalze, oder wenn in den Verbindungen der allgemeinen Formel I mindestens einer der Reste $R_1$, $R_2$ und $R_3$ die Bedeutung Hydroxy hat, Salze dieser Verbindungen mit pharmakologisch verträglichen Kationen,
dadurch gekennzeichnet, dass man

a) ein 1,3-Diaminopropan-2-ol der Formel II

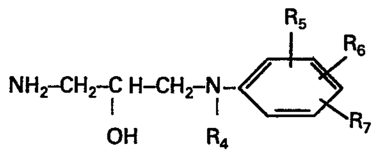

wobei $R_4$, $R_5$, $R_6$ und $R_7$ die oben angegebene Bedeutung haben, mit einem Benzoylderivat der Formel III

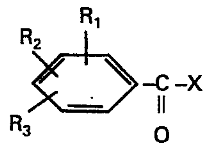

wobei $R_1$, $R_2$ und $R_3$ verätherte und/oder veresterte Hydroxygruppen der oben genannten Bedeutung sind und X ein reaktionsfähiger Säurerest, wie Halogen, eine niedermolekulare Alkoxygruppe oder O–CO–Y ist, worin Y eine niedermolekulare Alkoxygruppe bedeutet, ggf. in einem inerten Lösungsmittel bei Temperaturen zwischen – 10 °C und dem Siedepunkt des eingesetzten Lösungsmittels bei Normaldruck oder bei erhöhtem Druck umsetzt zur Verbindung der Formel I

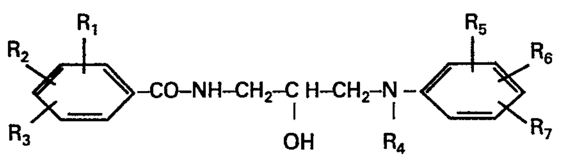

in welcher $R_1$, $R_2$ und $R_3$ verätherte und/oder veresterte Hydroxygruppen der oben genannten Bedeutung sind und $R_4$, $R_5$, $R_6$ und $R_7$ die obige Bedeutung haben,

b) gegebenenfalls einen oder mehrere der Substituenten $R_1$, $R_2$ und $R_3$ mit Bedeutung Benzyloxy oder Chlorbenzyloxy hydrogenolytisch zu den entsprechenden Hydroxygruppen spaltet,

c) gegebenenfalls Verbindungen mit den Substituenten $R_1$, $R_2$ oder $R_3$ mit Bedeutung Acetoxy oder Alkoxycarbonyloxy durch alkalische Hydrolyse in die entsprechenden Hydroxyverbindungen überführt,

d) gegebenenfalls einen oder mehrere der Substituenten $R_1$, $R_2$ und $R_3$ mit Bedeutung Hydroxy in die gewünschte Alkoxy-, Allyloxy-, Propargyloxy- oder in die gegebenenfalls substituierte Benzyloxygruppe der obigen Bedeutung umwandelt,

e) gegebenenfalls $R_4$ mit Bedeutung Wasserstoffatom in $R_4$ mit Bedeutung Alkyl umwandelt,

f) die isolierten freien Basen der Verbindungen der Formel I in die Säureadditionssalze überführt oder aus den Säureadditionssalzen die freien Basen gewinnt oder

g) gegebenenfalls die Verbindungen der Formel I mit pharmakologisch verträglichen Kationen zu den entsprechenden Metall-Oxidoverbindungen umsetzt, wenn mindestens einer der Reste $R_1$, $R_2$ und $R_3$ die Bedeutung Hydroxy hat.

10. Verfahren nach Anspruch 9, dadurch gekennzeichnet, dass man die Umsetzung der 1,3-Diaminopropan-2-ole der Formel II mit den Benzoylderivaten der Formel III in Gegenwart eines säurebindenden Reagenzes, nämlich Kaliumcarbonat, Natriumcarbonat, Natriumhydroxid, Kaliumhydroxid, Triäthylamin oder Pyridin, durchführt und gegebenenfalls das im Überschuss eingesetzte tertiäre Amin als Lösungsmittel verwendet.

11. Verfahren nach Anspruch 9, dadurch gekennzeichnet, dass man die hydrogenolytische Spaltung der Benzyloxygruppen mit Wasserstoff in Gegenwart eines Edelmetallkatalysators durchführt.

12. Verfahren nach Anspruch 11, dadurch gekennzeichnet, dass man als Edelmetallkatalysator Palladiumkohle einsetzt und die Umsetzung zwischen 15 und 50 °C in einem inerten Lösungsmittel durchführt.

13. Verfahren nach Anspruch 9, dadurch gekennzeichnet, dass man die alkalische Hydrolyse der Acetoxy- oder Alkoxycarbonyloxy-Gruppen in einem inerten Lösungsmittel zwischen 25 und 80 °C und in Inertgasatmosphäre durchführt.

14. Verfahren nach Anspruch 9, dadurch gekennzeichnet, dass man zur Einführung von Äthergruppen in den Benzoylring ein Alkalisalz der $N_1$-Hydroxybenzoylverbindungen in Gegenwart von inerten Lösungsmitteln mit einem Halogenalkan, Halogenalken, Halogenalkin oder Halogenbenzyl in Inertgasatmosphäre umsetzt.

15. Arzneimittel, bestehend aus einer oder mehreren Verbindungen gemäss Anspruch 1 und üblichen Träger und Hilfsstoffen.

**Revendications**

1. Nouveaux $N_1$-benzoyl-$N_2$-phényl-1,3-diaminopropan-2-ols de formule I:

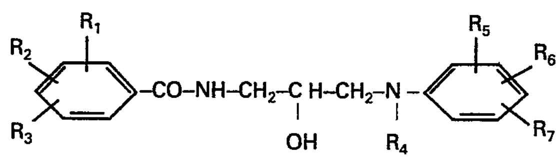

dans laquelle $R_1$, $R_2$ et $R_3$ peuvent être identiques ou différents et représentent chacun un groupe hydroxy, un groupe benzyloxy, un groupe chlorobenzyloxy ou un groupe alcoxy contenant 1 à 4 atomes de carbone, deux groupes voisins pouvant former un groupe méthylène-dioxy ou un groupe éthylène-dioxy, ou deux groupes méthoxy étant combinés avec un groupe allyloxy, un groupe propargyloxy, un groupe acétoxy ou un groupe alcoxycarbonyloxy dans lequel le groupe alcoxy peut contenir, au maximum, 2 atomes de carbone,

$R_4$ représente un groupe alkyle contenant 1 à 4 atomes de carbone, un groupe β-hydroxyéthyle, un groupe β-méthoxyéthyle ou un atome d'hydrogène,

$R_5$, $R_6$ et $R_7$ peuvent être identiques ou différents et représentent chacun un atome d'hydrogène, un

atome d'halogène, un groupe alkyle contenant 1 à 4 atomes de carbone, ou un groupe alcoxy contenant 1 à 4 atomes de carbone, deux groupes voisins pouvant former un groupe méthylène-dioxy ou un groupe éthylène-dioxy, ou encore un des radicaux $R_5$, $R_6$ et $R_7$ représente un groupe trifluorométhyle ou un groupe nitro,
ou, lorsque $R_1$, $R_2$ et $R_3$ ensemble représentent un groupe 3,4,5-triméthoxy et que $R_4$ est un groupe méthyle, au maximum deux des radicaux $R_5$, $R_6$ et $R_7$ représentent chacun un atome d'hydrogène, de même que leurs sels d'addition d'acide, ou lorsque, dans les composés de formule générale I, au moins un des radicaux $R_1$, $R_2$ et $R_3$ représente un groupe hydroxy, les sels de ces composés avec des cations pharmacologiquement compatibles.

2. $N_1$-(3,4,5-triméthoxybenzoyl)-$N_2$-A-1,3-diaminopropan-2-ol suivant la revendication 1, dans lequel A représente un groupe phényle, un groupe 2-fluorophényle, un groupe 3-fluorophényle, un groupe 4-fluorophényle, un groupe 2-chlorophényle, un groupe 2-méthylphényle, un groupe 3-methylphényle, un groupe 3,4-dichlorophényle, un groupe 3-chloro-2-méthylphényle, un groupe 2,6-diméthylphényle ou un groupe 2,4,6-triméthylphényle.

3. $N_1$-(3,4,5-triméthoxybenzoyl)-$N_2$-méthyl-$N_2$-A-1,3-diaminopropan-2-ol suivant la revendication 1, dans lequel A représente un groupe 4-fluorophényle, un groupe 4-chlorophényle, un groupe 4-bromophényle, un groupe 4-méthylphényle, un groupe 4-éthylphényle, un groupe 4-isopropylphényle, un groupe 4-trifluorométhylphényle, un groupe 3-méthoxyphényle, un groupe 3,4-dichlorophényle, un groupe 3,4-diméthoxyphényle ou un groupe 4-nitrophényle.

4. $N_1$-(3,4,5-triméthoxybenzoyl)-$N_2$-B-$N_2$-phényl-1,3-diaminopropan-2-ol suivant la revendication 1, dans lequel B représente un groupe éthyle, un groupe propyle, un groupe isopropyle ou un groupe β-hydroxyéthyle.

5. $N_1$-(3,4,5-triméthoxybenzoyl)-$N_2$-B-$N_2$-(4-chlorophényl)-1,3-diaminopropan-2-ol suivant la revendication 1, dans lequel B représente un groupe propyle ou un groupe β-méthoxyéthyle.

6. $N_1$-(3,4,5-triméthoxybenzoyl)-$N_2$-éthyl-$N_2$-(4-fluorophényl)-1,3-diaminopropan-2-ol.

7. $N_1$-C-$N_2$-méthyl-$N_2$-(4-fluorophényl)-1,3-diaminopropan-2-ol suivant la revendication 1, dans lequel C représente un groupe 2,3,4-triméthoxybenzoyle, un groupe 2,4,5-triméthoxybenzoyle, un groupe 3,4,5-triéthoxybenzoyle, un groupe 4-méthoxy-3,5-dibenzyloxybenzoyle, un groupe 3,4,5-tribenzyloxybenzoyle, un groupe 4-acétoxy-3,5-diméthoxybenzoyle, un groupe 4-éthoxycarbonyloxy-3,5-diméthoxybenzoyle, un groupe 4-hydroxy-3,5-diméthoxybenzoyle, un groupe 4-méthoxy-3,5-dihydroxybenzoyle, un groupe 3,4,5-trihydroxybenzoyle, un groupe 4-isopropoxy-3,5-diméthoxybenzoyle, un groupe 4-butoxy-3,5-diméthoxybenzoyle, un groupe 4-allyloxy-3,5-diméthoxybenzoyle, un groupe 4-propargyloxy-3,5-diméthoxybenzoyle, un groupe 4-(4-chlorobenzyloxy)-3,5-diméthoxybenzoyle, un groupe 2-méthoxy-4,5-méthylène-dioxybenzoyle, un groupe 4-méthoxy-2,3-éthylène-dioxybenzoyle, un groupe 4-éthoxy-2,3-éthylène-dioxybenzoyle, un groupe 3-méthoxy-4,5-éthylène-dioxybenzoyle, un groupe 3-éthoxy-4,5-éthylène-dioxybenzoyle.

8. $N_1$-C-$N_2$-méthyl-$N_2$-(4-chlorophényl)-1,3-diaminopropan-2-ol suivant la revendication 1, dans lequel C représente un groupe 4-méthoxy-3,5-dibenzyloxybenzoyle ou un groupe 4-méthoxy-3,5-dihydroxybenzoyle.

9. Procédé de préparation de $N_1$-benzoyl-$N_2$-phényl-1,3-diaminopropan-2-ols de formule I:

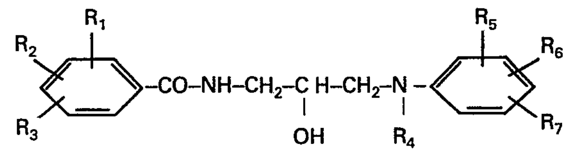

dans laquelle $R_1$, $R_2$ et $R_3$ peuvent être identiques ou différents et représentent chacun un groupe hydroxy, un groupe benzyloxy, un groupe chlorobenzyloxy ou un groupe alcoxy contenant 1 à 4 atomes de carbone, deux groupes voisins pouvant former un groupe méthylène-dioxy ou un groupe éthylène-dioxy, ou deux groupes méthoxy étant combinés avec un groupe allyloxy, un groupe propargyloxy, un groupe acétoxy ou un groupe alcoxycarbonyloxy dans lequel le groupe alcoxy peut contenir, au maximum, 2 atomes de carbone,
$R_4$ représente un groupe alkyle contenant 1 à 4 atomes de carbone, un groupe β-hydroxyéthyle, un groupe β-méthoxyéthyle ou un atome d'hydrogène,
$R_5$, $R_6$ et $R_7$ peuvent être identiques ou différents et représentent chacun un atome d'hydrogène, un atome d'halogène, un groupe alkyle contenant 1 à 4 atomes de carbon ou un groupe alcoxy contenant 1 à 4 atomes de carbone, deux groupes voisins pouvant former un groupe méthylène-dioxy ou un groupe éthylène-dioxy, ou un des radicaux
$R_5$, $R_6$ et $R_7$ représente un groupe trifluorométhyle ou un groupe nitro, ou, lorsque $R_1$, $R_2$ et $R_3$ ensemble représentent un groupe 3,4,5-triméthoxy et que $R_4$ est un groupe méthyle, au maximum deux des radicaux $R_5$, $R_6$ et $R_7$ représentent chacun un atome d'hydrogène, ainsi que de leurs sels d'addition d'acide, ou lorsque, dans les composés de formule générale I, au moins un des radicaux $R_1$, $R_2$ et $R_3$ représente un groupe hydroxy, des sels de ces composés avec des cations pharmacologiquement compatibles, caractérisé en ce que:
a) on fait réagir un 1,3-diaminopropan-2-ol de formule II:

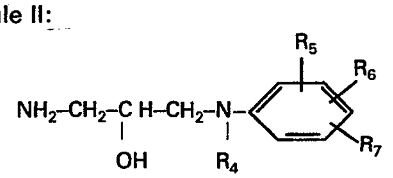

dans laquelle $R_4$, $R_5$, $R_6$ et $R_7$ ont les significations indiquées ci-dessus, avec un dérivé de benzoyle de formule III:

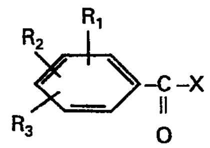

dans laquelle $R_1$, $R_2$ et $R_3$ représentent chacun un groupe hydroxy éthérifié et/ou estérifié ayant la signification mentionnée ci-dessus, tandis que X représente un radical acide réactif tel qu'un atome d'halogène, un groupe alcoxy de faible poids moléculaire ou un groupe O–CO–Y dans lequel Y représente un groupe alcoxy de faible poids moléculaire, éventuellement dans un solvant inerte, à des températures comprises entre −10 °C et le point d'ébullition du solvant utilisé, sous pression normale ou sous pression élevée, pour obtenir un composé de formule I

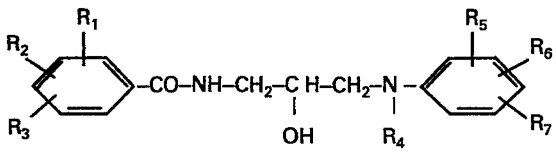

dans laquelle $R_1$, $R_2$ et $R_3$ représentent chacun un groupe hydroxy éthérifié et/ou estérifié ayant la signification indiquée ci-dessus, tandis que $R_4$, $R_5$, $R_6$ et $R_7$ ont les significations indiquées ci-dessus,
b) on soumet éventuellement un ou plusieurs des substituants $R_1$, $R_2$ et $R_3$ représentant un groupe benzyloxy ou un groupe chlorobenzyloxy à une scission par voie hydrogénolytique pour obtenir les groupes hydroxy correspondants,
c) on transforme éventuellement des composés comportant les substituants $R_1$, $R_2$ et $R_3$ représentant un groupe acétoxy ou un groupe alcoxycarbonyloxy, en composés hydroxy correspondants par hydrolyse alcaline,
d) on transforme éventuellement un ou plusieurs des substituants $R_1$, $R_2$ et $R_3$ représentant un groupe hydroxy, en un groupe alcoxy, un groupe allyloxy ou un groupe propargyloxy désiré, ou encore en un groupe benzyloxy éventuellement substitué ayant la signification indiquée ci-dessus,
e) on transforme éventuellement le radical $R_4$ représentant un atome d'hydrogène, en un radical $R_4$ représentant un groupe alkyle,
f) on transforme les bases libres isolées des composés de formule I en sels d'addition d'acide, ou on obtient les bases libres à partir des sels d'addition d'acide, ou encore
g) on fait éventuellement réagir les composés de formule I avec des cations pharmacologiquement compatibles pour obtenir les oxydo-composés métalliques correspondants lorsqu'au moins un des radicaux $R_1$, $R_2$ et $R_3$ représente un groupe hydroxy.

10. Procédé suivant la revendication 9, caractérisé en ce qu'on effectue la réaction des 1,3-diaminopropan-2-ols de formule II avec les dérivés de benzoyle de formule III en présence d'un réactif fixateur d'acide, notamment le carbonate de potassium le carbonate de sodium, l'hydroxyde de sodium, l'hydroxyde de potassium, la triéthylamine ou la pyridine et on utilise éventuellement, comme solvant, l'amine tertiaire employée en excès.

11. Procédé suivant la revendication 9, caractérisé en ce qu'on effectue la scission hydrogénolytique des groupes benzyloxy avec de l'hydrogène en présence d'un catalyseur d'un métal noble.

12. Procédé suivant la revendication 11, caractérisé en ce que, comme catalyseur d'un métal noble, on utilise le charbon palladié et on effectue la réaction dans un solvant inerte à une température comprise entre 15 et 50 °C.

13. Procédé suivant la revendication 9, caractérisé en ce qu'on effectue l'hydrolyse alcaline des groupes acétoxy ou alcoxycarbonyloxy dans un solvant inerte, à une température comprise entre 25 et 80 °C, ainsi que sous une atmosphère d'un gaz inerte.

14. Procédé suivant la revendication 9, caractérisé en ce que, pour introduire des groupes éthers dans le noyau benzoyle, on fait réagir un sel alcalin des composés de $N_1$-hydroxybenzoyle avec un halogénalcane, un halogénalcène, une halogénalcyne ou un halogénobenzyle en présence de solvants inertes et sous une atmosphère d'un gaz inerte.

15. Médicaments constitués d'un ou de plusieurs composés suivant la revendication 1, ainsi que de substances auxiliaires et de supports habituels.

**Claims**

1. New $N_1$-benzoyl-$N_2$-phenyl-1,3-diaminopropan-2-ols of the formula I

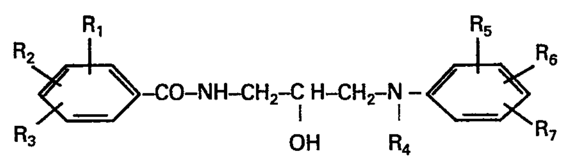

in which $R_1$, $R_2$ and $R_3$ can be the same and different and represent hydroxyl groups, benzyloxy, chlorobenzyloxy groups or alkoxy groups with 1 to 4 C atoms, in which two adjacent groups can form a methylenedioxy or ethylenedioxy group, or in which two methoxy groups are combined with an allyloxy, propargyloxy, acetoxy or alkoxycarbonyloxy group, in which the alkoxy group can have at most 2 C atoms,
$R_4$ is an alkyl group with 1 to 4 C atoms, a β-hydroxyethyl or β-methoxyethyl group or a hydrogen atom,
$R_5$, $R_6$ and $R_7$ can be the same or different and hydrogen or halogen atoms, alkyl groups with 1 to 4 C atoms, alkoxy groups with 1 to 4 C atoms, in which two adjacent groups can form a methylenedioxy or ethylenedioxy group, or one of the radicals $R_5$, $R_6$ and $R_7$ is a trifluoromethyl or a nitro group or, if $R_1$, $R_2$ and $R_3$ together denote 3,4,5-trimethoxy and $R_4$ is a methyl group, not more than two of the radicals $R_5$, $R_6$ and $R_7$ denote a hydrogen atom, and acid addition salts thereof, or if, in the compounds of the general formula I, at

least one of the radicals $R_1$, $R_2$ and $R_3$ denotes a hydroxyl group, salts of these compounds with pharmacologically acceptable cations.

2. An $N_1$-(3,4,5-trimethoxybenzoyl)-$N_2$-A-1,3-diaminopropan-2-ol according to Claim 1, in which A is a phenyl, 2-fluorophenyl, 3-fluorophenyl, 4-fluorophenyl, 2-chlorophenyl, 2-methylphenyl, 3-methylphenyl, 3,4-dichlorophenyl, 3-chloro-2-methylphenyl, 2,6-dimethylphenyl or 2,4,6-trimethylphenyl radical.

3. An $N_1$-(3,4,5-trimethoxybenzoyl)-$N_2$-methyl-$N_2$-A-1,3-diaminopropan-2-ol according to Claim 1, in which A is a 4-fluorophenyl, 4-chlorophenyl, 4-bromophenyl, 4-methylphenyl, 4-ethylphenyl, 4-isopropylphenyl, 4-trifluoromethylphenyl, 3-methoxyphenyl, 3,4-dichlorophenyl or 3,4-dimethoxyphenyl or 4-nitrophenyl radical.

4. An $N_1$-(3,4,5-trimethoxybenzoyl)-$N_2$-B-$N_2$-phenyl-1,3-diaminopropan-2-ol according to Claim 1, in which B is an ethyl, propyl, isopropyl or β-hydroxyethyl radical.

5. An $N_1$-(3,4,5-trimethoxybenzoyl)-$N_2$-B-$N_2$-(4-chlorophenyl)-1,3-diaminopropan-2-ol according to Claim 1, in which B is a propyl or β-methoxyethyl radical.

6. $N_1$-(3,4,5-trimethoxybenzoyl)-$N_2$-ethyl-$N_2$-(4-fluorophenyl)-1,3-diaminopropan-2-ol.

7. An $N_1$-C-$N_2$-methyl-$N_2$-(4-fluorophenyl)-1,3-diaminopropan-2-ol according to Claim 1, in which C is a 2,3,4-trimethoxybenzoyl, 2,4,5-trimethoxybenzoyl, 3,4,5-triethoxybenzoyl, 4-methoxy-3,5-dibenzyloxybenzoyl, 3,4,5-tribenzyloxybenzoyl, 4-acetoxy-3,5-dimethoxybenzoyl, 4-ethoxycarbonyloxy-3,5-dimethoxybenzoyl, 4-hydroxy-3,5-dimethoxybenzoyl, 4-methoxy-3,5-dihydroxybenzoyl, 3,4,5-trihydroxybenzoyl, 4-isopropoxy-3,5-dimethoxybenzoyl, 4-butoxy-3,5-dimethoxybenzoyl, 4-allyloxy-3,5-dimethoxybenzoyl, 4-propargyloxy-3,5-dimethoxybenzoyl, 4-(4-chlorobenzyloxy)-3,5-dimethoxybenzoyl, 2-methoxy-4,5-methylenedioxybenzoyl, 4-methoxy-2,3-ethylenedioxybenzoyl, 4-ethoxy-2,3-ethylenedioxybenzoyl, 3-methoxy-4,5-ethylenedioxybenzoyl, (or) 3-ethoxy-4,5-ethylenedioxybenzoyl radical.

8. An $N_1$-C-$N_2$-methyl-$N_2$-(4-chlorophenyl)-1,3-diaminopropan-2-ol according to Claim 1, in which C is a 4-methoxy-3,5-dibenzyloxybenzoyl or 4-methoxy-3,5-dihydroxybenzoyl radical.

9. Process for the preparation of $N_1$-benzoyl-$N_2$-phenyl-1,3-diaminopropan-2-ols of the formula I

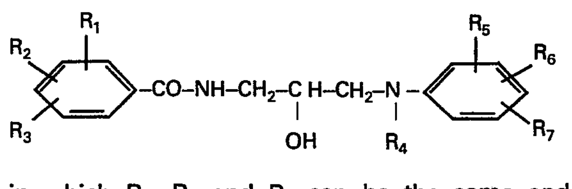

in which $R_1$, $R_2$ and $R_3$ can be the same and different and represent hydroxyl groups, benzyloxy, chlorobenxyloxy groups or alkoxy groups with 1 to 4 C atoms, in which two adjacent groups can form a methylenedioxy or ethylenedioxy group, or in wich two methoxy groups are combined with an allyloxy, propargyloxy, acetoxy or alkoxycarbonyloxy group, in which the alkoxy group can have at most 2 C atoms,

$R_4$ is an alkyl group with 1 to 4 C atoms, a β-hydroxyethyl or β-methoxyethyl group or a hydrogen atom,

$R_5$, $R_6$ and $R_7$ can be the same of different and hydrogen or halogen atoms, alkyl groups with 1 to 4 C atoms, alkoxy groups with 1 to 4 C atoms, in which two adjacent groups can form a methylenedioxy or ethylenedioxy group, or one of the radicals

$R_5$, $R_6$ and $R_7$ is a trifluoromethyl or a nitro group or, if $R_1$, $R_2$ and $R_3$ together denote 3,4,5-trimethoxy and $R_4$ is a methyl group, not more than two of the radicals $R_5$, $R_6$ and $R_7$ denote a hydrogen atom, and acid addition salts thereof, or if, in the compounds of the general formula I, at least one of the radicals $R_1$, $R_2$ and $R_3$ denotes a hydroxyl group, salts of these compounds with pharmacologically acceptable cations. characterised in that

a) a 1,3-diaminopropan-2-ol of the formula II

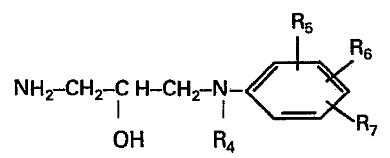

in which $R_4$, $R_5$, $R_6$ and $R_7$ have the meanings indicated above is reacted with a benzoyl derivative of the formula III

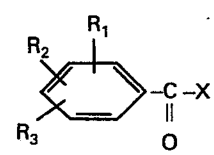

in which $R_1$, $R_2$ and $R_3$ are etherified and/or esterified hydroxyl groups of the meanings given above and X is a reactive acid radical, such as halogen, a low-molecular alkoxy group or O–CO–Y where Y represents a low-molecular alkoxy group, optionally in an inert solvent at a temperature between −10 °C and the boiling point of the solvent employed under normal pressure or under elevated pressure, to give a compound of the formula I

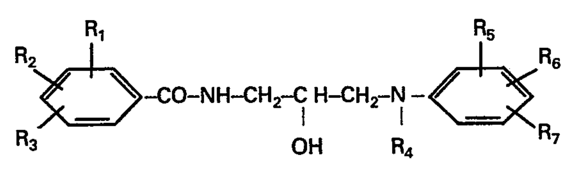

in which $R_1$, $R_2$ and $R_3$ are etherified and/or esterified hydroxyl groups of the meanings given above and $R_4$, $R_5$, $R_6$ and $R_7$ have the above meanings,

b) optionally, one or more of the substituents $R_1$, $R_2$ and $R_3$ which denotes a benzyloxy or chlorobenzyloxy group is split hydrogenolytically to the corresponding hydroxyl group;

c) optionally, compounds with the substituents $R_1$, $R_2$ and $R_3$ denoting acetoxy or alkoxycarbonyloxy are converted by alkaline hydrolysis to the corresponding hydroxyl compounds,

d) optionally, one or more of the substituents $R_1$, $R_2$ and $R_3$ denoting hydroxy is converted to the desired alkoxy, allyloxy, propargyloxy group or to the optionally substituted benzyloxy group of the meaning given above,

e) optionally, $R_4$ denoting a hydrogen atom is converted to $R_4$ denoting alkyl,

f) the isolated free base of the compound of formula I is converted to the acid addition salt, or the free base is obtained from the acid addition salt, or

g) optionally, the compound of formula I is reacted with pharmacologically acceptable cations to give the corresponding metal-oxide compounds, if at least one of the radicals $R_1$, $R_2$ and $R_3$ denotes hydroxy.

10. A process according to Claim 9, characterised in that the reaction of the 1,3-diaminopropan-2-ols of formula II with the benzoyl derivatives of formula III is carried out in the presence of an acid-binding reagent, namely potassium carbonate, sodium carbonate, sodium hydroxide, potassium hydroxide, triethylamine or pyridine, and optionally an excess of the tertiary amine employed is used as the solvent.

11. A process according to Claim 9, characterised in that hydrogenolytic splitting of the benzyloxy groups is carried out with hydrogen in the presence of a noble metal catalyst.

12. A process according to Claim 11, characterised in that the noble metal catalyst is palladium-on-charcoal and the reaction is carried out between 15 and 50 °C in an inert solvent.

13. A process according to Claim 9, characterised in that the alkaline hydrolysis of the acetoxy or alkoxycarbonyloxy groups is carried out in an inert solvent between 25 and 80 °C and in an inert gas atmosphere.

14. A process according to Claim 9, characterised in that in order to introduce ether groups into the benzoyl ring, an alkali salt of the $N_1$-hydroxybenzoyl compounds is reacted in the presence of inert solvents with a haloalkane, haloalkene, haloalkyne or halobenzyl in an inert gas atmosphere.

15. A pharmaceutical composition comprising one or more compounds according to Claim 1 and conventional carriers and auxiliaries.